# EUROPEAN PATENT SPECIFICATION

(11) **EP 4 477 166 B1**
(45) Date of publication and mention of the grant of the patent: **04.11.2026**
(21) Application number: 24210285.3
(22) Date of filing: 04.09.2018
(51) Int. Cl.: A61B 17/72, A61B 17/68, A61B 17/86

(54) **INTRAMEDULLARY THREADED NAIL FOR RADIAL CORTICAL FIXATION**
GEWINDEMARKNAGEL ZUR RADIALEN KORTIKALISFIXIERUNG
CLOU FILETÉ INTRAMÉDULLAIRE POUR FIXATION CORTICALE RADIALE

(30) Priority: 05.09.2017 US 201762554123 P
(43) Date of publication of application: 18.12.2024
(62) Document number(s) of the earlier application(s) in accordance with Art. 76 EPC: 18854272.4 / 3 678 565
(73) Proprietor: ExsoMed Corporation, Hillsboro, OR 97124 (US)
(72) Inventor: CHAMPAGNE, Lloyd P., Phoenix, Arizona 85018 (US); ZOLDOS, Jozef, Paradise Valley, Arizona 85253 (US); BRIGANTI, Richard T., Bala Cynwyd, Pennsylvania 19004 (US); LEITHER, Andrew J., Akron, Ohio 44333 (US)
(74) Representative: Boult Wade Tennant LLP

(56) References cited:
- US-A1- 2013 030 475
- US-A1- 2013 274 814

## Description

### FIELD OF THE INVENTION

The present invention relates to a device and system for fixation of fractures and non-unions of small bones and other small bone fragments, and more particularly to a threaded nail with a robust length and a headless leading end with a torque driving recess and a trailing end with a cutting tip and longitudinal cutting flutes, and an intermediate stepped diameter with cutting flutes at the transition point, Optionally, the device includes a cannulation along the central longitudinal axis of the nail. This intramedullary nail provides for full shaft-length and internally circumferential cortical fixation in particular for use in the metacarpals and metatarsals. Thus, the implant achieves at least a frictional fit circumferentially with the internal cortex for at least 60% +/-15% of the intramedullary canal length by means of engagement with a penetrating supplemental spiral thread along at least 80% +/-15% of the length of the implant.

### BACKGROUND OF THE INVENTION

Trauma to the mid-hand and mid-foot can have life-changing effects for the victim as a result of loss of function, disfiguration, and even the lengthy and onerous treatment protocols. It's hard and sometimes impossible to do without the use of a foot or hand, and in particular, when one is living "hand to mouth".

Current methods of treatment include internal fixation with surgery, like plating systems and wire fixation or in the case of non-displaced fractures, casting the hand or foot without surgical intervention. These treatments involve a substantial period, i.e., several weeks or months, in which the injured hand or foot is immobilized. However, serious problems with adhesions, loss of muscle, proprioception and nerve health, can result from dis-use for these periods of time. Moreover, beyond the side effects of the treatment protocol, there are problems with compliance and with the issues of daily living that result from not being able to use a hand or foot for these prolonged periods of time.

While it is possible for hand and foot accidents to occur during various activities, including traffic accidents, firearm mis-fire, and extreme sport activities, there is a greater correlation of the risk for such traumatic events in manual and low wage jobs. Thus, the incidence and the effects of such accidents are skewed to jobs on the lower end of the payscale and may often involve workers who are already at economic risk. Thus, victims of hand or foot trauma will often either resort to non-compliance involving the early use of the injured limb, or in some instances will suffer the loss of income, including the loss of a job altogether, which sets off a spiraling decline in the ability to "make ends meet", expanding the risk from the injury to the loss of house and home and family stability.

Finally, it should be noted that the hand is paramount to every aspect of our humanity and is instrumental in dictating and defining our relationship to our world. Injury to the hand hinders that relationship and our perception of our role in that world, especially in cases where the hand is disfigured, or appears to be disabled. Our hands are constantly in view to ourselves and to the world, and debilitating scars or irregularities such as scars, and even the Frankenstein-like outlines of surgical hardware on the mid-hand, are visible and painful reminders of a traumatic event and its aftermath.

The prior art orthopedic implants for use in small bones in the arms and legs have a set of criteria that differs from those of the long bones and large joints. These bones, (generally in the elbow and below and the knee and below), tend to be smaller in diameter and present a differing ratio of cortical to cancellous bone, as well as the issues of covering soft tissue and a relatively large nexus of tendons, ligaments and nerves, to complicate surgical techniques. The present invention is intended to answer these issues, and to provide a solution to surgical fixation of broken or fractured small bones through a threaded nail intended for intramedullary fixation and most importantly, allowing a swift return to daily living, and reduced disability, pain, and rehabilitation.

One typical small bone implant location is the hand or foot, and in particular in the metacarpals and metatarsals. The structure of the hand is composed of the scaphoid and other carpal bones which meet with the radius and the ulna at the wrist joint, the metacarpals, and phalanges. The back of the hand includes the metacarpals, and a metacarpal connects each finger and thumb to the hand, while the fingers and thumb are formed of bones called phalanges. The connection of the phalanges to the metacarpals is called a "knuckle" joint or metacarpophalangeal joint (MCP joint), and acts like a hinge when the fingers or thumb are bent. In each finger, there are three phalanges that are separated by two joints called the interphalangeal joints (IP joints). The proximal IP joint (PIP joint) is the one closest to the MCP joint. The other joint closest to the end of the finger is the distal IP joint (DIP joint). The thumb just has one IP joint. The joints are covered on the ends with articular cartilage. The foot has an analogous structure, with the talus interfacing between the tibia and the fibula on the leg, and the calcaneus on the foot, and the mid-foot being comprised of the tarsals, including the cuneiforms, (i.e., the cuboid and the navicular bones,) the metatarsals, and the phalanges.

Damage to these bones of the extremities may occur as a result of a sprain or fracture or trauma and typically following reduction to re-align a damaged bone, it should be stabilized in position to allow it to heal in that position.

In the past, a surgical procedure to stabilize these broken bones has involved drilling a pilot hole and insertion of a k-wire or smooth nail along the length of the bone to hold it in position while the bone heals. An opening is first formed in the metacarpal bone, wherein the opening extends through the fracture and the nail is positioned by wedging it in the opening through the soft cancellous intermediate bone so that the nail provides stability for the parts of the bone on either side of the fracture. After a certain period, a second surgery is required to remove the nail from the bone. Problems with this procedure are that, because it is not anchored in the bone and in fact, mostly interfaces with the spongy central bone pulp, it can migrate through the metacarpal bone and into surrounding tissue. Sometimes this can result in damage to soft tissue, such as a severed or damaged tendon or cartilage, and/or cause pain. Another problem with the nail is that, because it can migrate, a second surgery is required to remove it. Additionally, the proximal end of pins and nails can cause tendon irritation, tendon rupture or skin irritation and infection.

One theoretical solution to this problem is to insert a screw into the bone however, the torque required to place a screw into the length of a metacarpal bone (which is a relatively thin, delicate but brittle bone) is high. Such a procedure would be lengthy, and there would be a possibility of bone damage, or damage to the driving head of the screw, which could prevent complete insertion into the opening formed in the bone. Current screws are not designed specifically for intramedullary placement. For instance, the current screws are frequently not long enough, nor do they account for the narrowed waist of the metacarpal or metatarsal bones.

Thus, the present invention is intended for fixation of intra-articular and extra-articular fractures and non-unions of small bones and small bone fragments; including for example, arthrodesis of small joints; bunionectomies and osteotomies, including scaphoid and other carpals bones, metacarpals, tarsals, metatarsals, patella, ulnar, styloid, capitellum, radial head and radial styloid, and is preferably intended for intramedullary fixation of metacarpal and phalangeal fractures to provide surgeons with a reliable solution through a simple approach. The robust length offering, (more than 3.5 centimeters, and preferably more than 4 centimeters, or 5 centimeters +/- 1 or 0.5 centimeters, or even up to 7.5 centimeters), with large length to diameter ratio (more than 15:1, and preferably more than 20:1 ), and with a differential diameter (having a leading length at a smaller diameter of 3-5mm, and a trailing length towards the nail head, of from 0.4-2mm smaller, with good examples of 3.2/3.6 or 4.0/4.5), accurately fits native metacarpal and phalangeal locations to create a strong, stable fixation and precise reduction. Moreover, the invention is intended to fill the intramedullary canal along substantially all its length (i.e., more that 45%, or preferably more than 50%, and preferably more than 60%, or 70% and up to 80% or 98% by length). The design includes a leading, meaning away from the nail end, section having a smaller outer diameter of the root and also of the thread crest at that section, but a relatively constant internal cannulation where the leading section is from 40-60% of the length. The larger diameter section reinforces the area next to the torque receiving end of the nail so that the greater material allows transmission of the torque through the device, and the cutting flutes in the transition section between the dual diameters helps to keep the smaller leading portion from collapsing on itself. Thus, the device is designed to allow both insertion and removal by screwing or unscrewing the implant into the intramedullary canal.

In addition, the invention is unique as it is designed to capture internal cortical bone so that not only does the nail extend a substantial distance of the length of the shaft (at least 60% including penetration past the isthmus), it also is used so that is achieves "radial" or "circumferential" fixation within the canal, and preferably, achieves 360° internal circumferential cortical fixation. This is novel for the metacarpal application since the metacarpals (and metatarsals) include a narrowed waist area, the "isthmus" of the metacarpal (and metatarsals). Past screws and intramedullary implants are not designed to engage with sufficient length or to grab the internal walls of the canal circumferentially over substantial distance of the length, but the current design includes a stepped diameter with a leading cutting tip and an intermediate cutting feature that allows the invention to ream the canal to size as it is advanced through the canal. Thus, the canal is prepped for circumferential cortical fixation, which is unique to the present invention and refers to the use of an implant that includes a surface that is fixed to the internal cortical wall or of the intramedullary shaft, in particular of the metacarpal of the metatarsal. The nail is intended to avoid applying a compressive force along the length of the nail, and can be used in instances in which there is a void in the bone or missing bone fragments and nail stabilizes the remaining bone fragments with a gap to restore the length of the metacarpal. The nail is dual diameter with both leading-diameter and trailing-diameter sections substantially cylindrical and, importantly, not tapered or conical to permit the device to maintain contact with the hole in the canal during removal and to ease removal by unscrewing the nail.

The present invention can be used in a procedure that lets the patient return to work in a short period of time, from two weeks to the next day, including jobs ranging from manual labor, care-giver, professional musician, artist, and professional athlete. This return to functional living, and the decrease in down-time, and rehabilitation and risk to daily living is a huge societal saving.

US 2013/274814 A1 describes a bone joining device suitable for joining a first bone piece to a second bone piece. Also provided is a method of joining a first bone piece with a second bone piece in a living mammal. The method comprises inserting the above bone joining device between the first bone piece and the second bone piece.

### SUMMARY OF THE INVENTION

The present invention solves the problems associated with repairing a metacarpal or metatarsal fracture or dislocation by providing a device that is a unique combination of a nail and a screw, which can be inserted into the bone without damaging the bone because it is sized and shaped to achieve circumferential intramedullary cortical fixation and to support the bone from the inside, (even when there's a bone gap of from 0 up to 2, 2.5, or even 3cm along the length of a damaged metacarpal), to fit the narrowed portion of these bones by providing at least one stepped diameter and a fluted transition between the diameters, by providing a headless design with a uniform lead(s) which avoids compression and which can be seated below the surface of the bone to avoid the irritation that can result from a proud portion; by providing for a cannulated surgical technique with an intramedullary implant designed to minimize soft tissue, cartilage and vascular damage upon insertion; and to facilitate early, active mobilization post-operative protocols for accelerated healing and earlier return to work.

Further, as the device achieves radial internal fixation, the device is secured within the bone, which eliminates migration and eliminates the need for a second operation to remove the device. Thus, the device provides a cannulated cylindrical core having an outer exterior surface along its length, (from 35 to 75, or even as long as lengths to 120 or 130 mm) that defines an inner diameter (minor diameter) relative to the lateral edge of a threaded portion which defines an outer diameter (major diameter). The design includes one or more stepped portion which allow for an increase in the trailing portion of at least the outer diameter or the inner diameter, and more preferably of both the outer diameter and the inner diameter. The transition area also includes longitudinal cutting flutes (2-5 of 3 to 10mm length) to account for this increase at a length of the nail (i.e., from 25% to 75%, and preferably 35% to 60%, of the distance of the length from the trailing end) which allows a clearance of the narrowed or "waisted" portion of the bone. The cannulation is generally a constant diameter (for ease of manufacture) through the length of the nail, but could include a dual diameter which might include an over grind at the larger diameter or trailing end of the nail which includes the torque driving feature, such as an internal or external hexagon or hexalobe configuration.

The device includes a leading portion that has 2-5 cutting teeth on the beveled leading tip, and along the long side, also includes additional longitudinal flutes, which, as are the other flutes, intended to provide for reaming away of the cancellous intramedullary material and for scoring of the inner surface of the intramedullary channel, as well as a place for additional cancellous material to reside during surgery.

The thread defined between the outer diameter and the inner diameter can suitably include a right handed single start thread with a pitch of from 3 to 5mm, and preferably at least 4.0mm+/-0.25, or a dual lead thread of the same lead value and a pitch value half of the lead value with a secondary thread having a lower height, i.e., from 40% to 75% or 50% +/- 5% of the height of the primary thread. The leading and following flanks of the threads together form an angle of 5° to 60°, and preferably 15° ± 10°, and are connected by a thread crest of length 0.05 to 0.2mm which is separated from the root diameter by a thread depth of 0.2mm to 0.8mm.

At its first end, or trailing end, the device includes a driving recess capable of being driven by a suitable driver into the opening, such as a hexalobe or a T10 drive recess, to generate significant torque to bite into the cortical bone and avoid harm to the lower material narrower leading end of the nail.

### BRIEF DESCRIPTION OF THE DRAWINGS

FIG. 1 is a top side view of a device in accordance with the invention;
FIG. 2 is a side view of the device of FIG. 1;
FIG. 3 is a cross-section of the device of FIG. 1 taken along line A-A;
FIG. 4 is a detail of FIG. 3 showing the thread characteristics and a detail of the thread shape;
FIG. 5 is an end view of the proximal view of the device of FIG. 1;
FIG. 6 is an end view of the distal tip of the device of FIG. 1;
FIG. 7 is a side view detail of the transition section of the blank from which the invention is made and showing the intermediate cutting flutes of FIG. 1;
FIG. 8 is a side view detail of the distal end of the blank from which the invention is made and showing the distal cutting flutes of FIG. 1;
FIG. 9 is a top view of a skeleton of a hand showing the implant of the invention in place in a fractured metacarpal;
FIG. 10 is an illustration of the step of measuring a metacarpal for use with a technique which does not form part of the invention as claimed;
FIG. 11 is an illustration of the step of inserting a guide wire in a retrograde fashion to anatomically reduce the fracture fragments in accordance with the technique
FIG. 12 is an illustration of the entry point of the guide wire of FIG 11 in the dorsal third of the metacarpal head;
FIG. 13 is an illustration of the step of drilling by passing a cannulated drill over the guide wire;
FIG. 14 is an illustration of the step of inserting the implant in the metacarpal in accordance with a surgical technique which does not form part of the invention as claimed;
FIG. 15. is a side view of a second embodiment of the device of FIG. 1;
FIG. 16 is a cross-section of the device of FIG. 15 taken along line C-C;
FIG. 17 is a detail of FIG. 16 showing the thread characteristics and a detail of the thread shape;
FIG. 18 is a side view of the blank from which the invention is made and showing the transition section, the intermediate cutting flutes and distal cutting flutes of FIG. 15;
FIG. 19 is an end view of the proximal view of the device of FIG. 15; and
FIG. 20 is an end view of the distal tip of the device of FIG. 15.

### DETAILED DESCRIPTION OF THE INVENTION

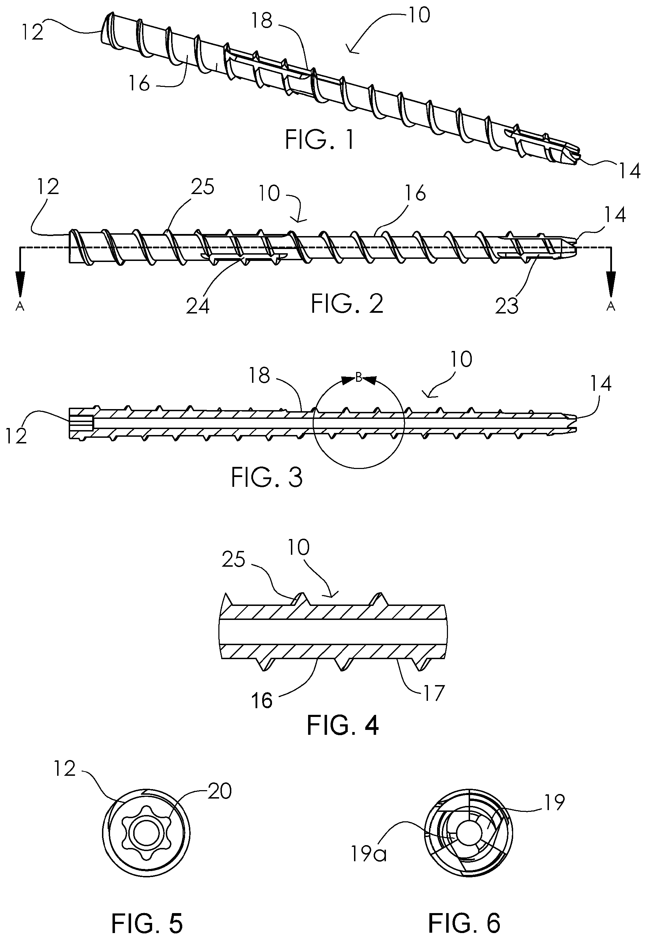

FIG. 1 shows an exemplary embodiment 10 of the threaded intramedullary nail of the present invention. The nail 10 may be formed of any suitable biocompatible material, such as surgical grade stainless steel, titanium, alloys of nickel and chromium, nitinol, PEEK, hydroxyapatite, bio-glass or other bio compatible materials or combinations of these materials. The nail 10 has a first end, or proximal end, 12, a second end, or distal end, 14, a shaft 16 with an outer surface 17, and a center portion 18 between first end 12 and second end 14. A cutting end 19 with a bevel and two, three or four teeth 19a is provided at the trailing end 14 (which is distal relative to the torque driving recess but is implanted in the proximal portion of the metacarpal) and a driving surface 20 in a drive recess 22 is formed in the top of first end 12.

The cutting point 19 helps to cut through any bone left behind when the bone is drilled to receive device 10, and further include 2-5 equally radially spaced longitudinal cutting flutes 23 which extend lengthwise in the exterior surface and through the threads of the nail 10. The driving surface 20 in this embodiment has a hexalobe drive configuration, although any suitable driving configuration may be used. Other driving configurations that may be used include slotted, Pozidriv, Robertson, tri-wing, Torq-Set, Spanner Head, Triple Square and hex head.

Extending length wise in outer surface 17, preferably along the longitudinal axis of shaft 16, are grooves or cutting flutes 24. As used herein, "extending length wise" means that each groove 24 is elongated and extends along the shaft with one end of the groove nearer the first end 12 and the opposite end of the groove nearer the second end 14, but grooves 24 may be formed at an angle and not necessarily formed along the longitudinal axis shaft 16, although that is preferred. Grooves 24 preferably have edges that assist in boring the device 10 into and anchoring device 10 in the opening in a bone. Grooves 24 also may capture some debris left behind from the bone drilling process to create the opening created when device 10 is positioned into the opening.

The shaft 18 of the nail 10 of the present invention includes at least two sections of differing diameters, for example, a proximal section that extends for a length of from about 15% to 30% of the length of the device, and which is located at from 25% to 75%, and preferably at 55-65% of the distance of the length from the distal end of the device 10. Further, the device provides a cannulated cylindrical core or shaft 18 having an outer exterior surface 17 along its length, (which is provided in 5mm increments from 35 to 75 mm) that defines an inner diameter relative to the lateral edge of a threaded portion which defines an outer diameter. The design includes one or more stepped portion which allow for a 0.25-1.0mm increase in the proximal portion of at least the outer diameter or the inner diameter, and more preferably of both the outer diameter and the inner diameter. The transition area also includes longitudinal cutting flutes (2-5 of 3 to 10 mm length) to account for this increase at a length of the nail (i.e., from 25% to 75%, and preferably from 50% to 60%, of the distance of the length from the proximal end) which allows a clearance of the narrowed or "waisted" portion of the bone.

The device includes a thread 25 which is defined between the outer diameter and the inner diameter and can suitably include a right-handed single start thread with a pitch of from 3 to 5mm, and preferably at least 4.0+/-0.25mm with a similar lead value.

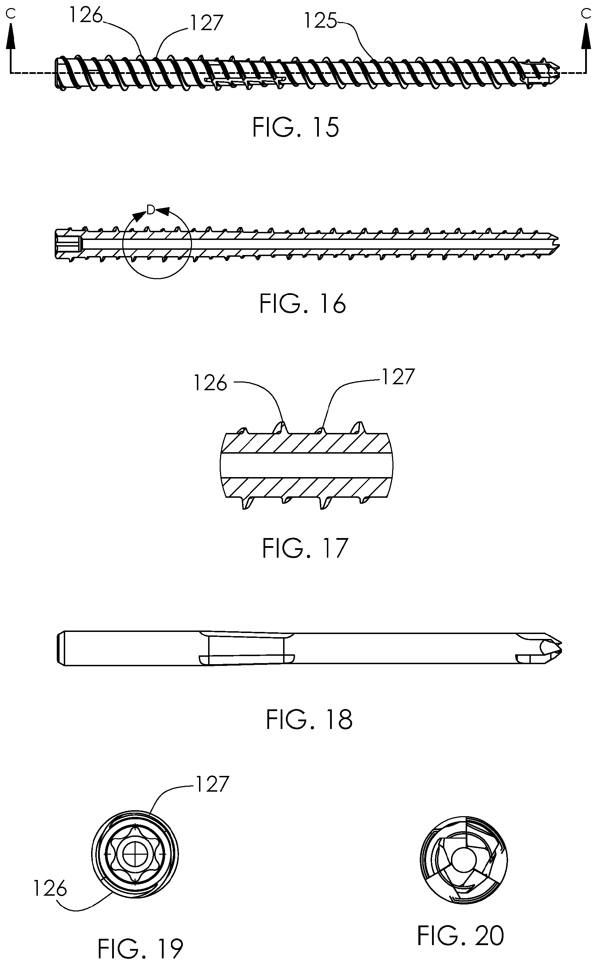

Alternatively, as is shown in FIGS. 15, 16 and 17, the device may include a double lead thread 125 having a first thread 126 and a second thread 127 of the same lead value with the second thread 127 having a height that is approximately 50% of the height of the first thread 126.

The leading and following flanks of the threads together form an angle of 5° to 60°, and preferably 15 ± 10°, and are connected by a thread crest of length 0.05 to 0.2mm which is separated from the root diameter by a thread depth of 0.2mm to 0.8mm.

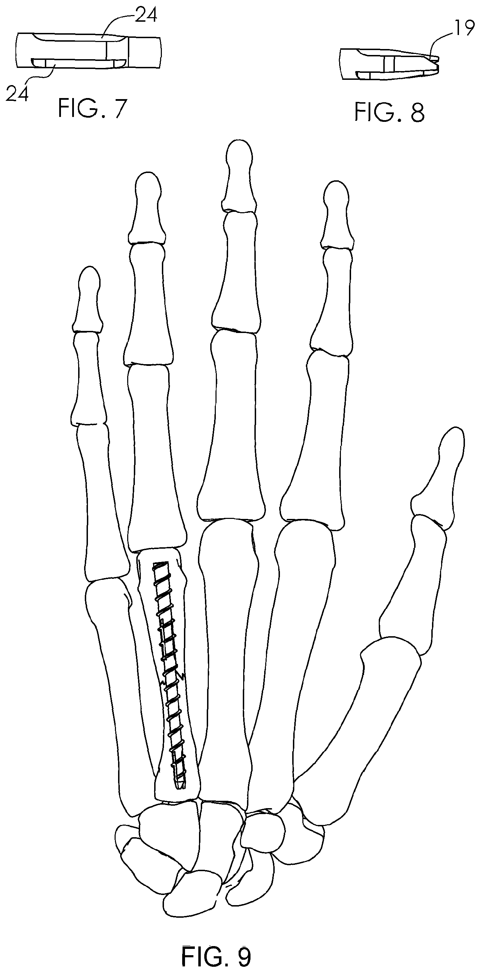

FIG. 9 illustrates a nail 10 in accordance with the present invention in position in a fourth metacarpal and securing a reduced fracture.

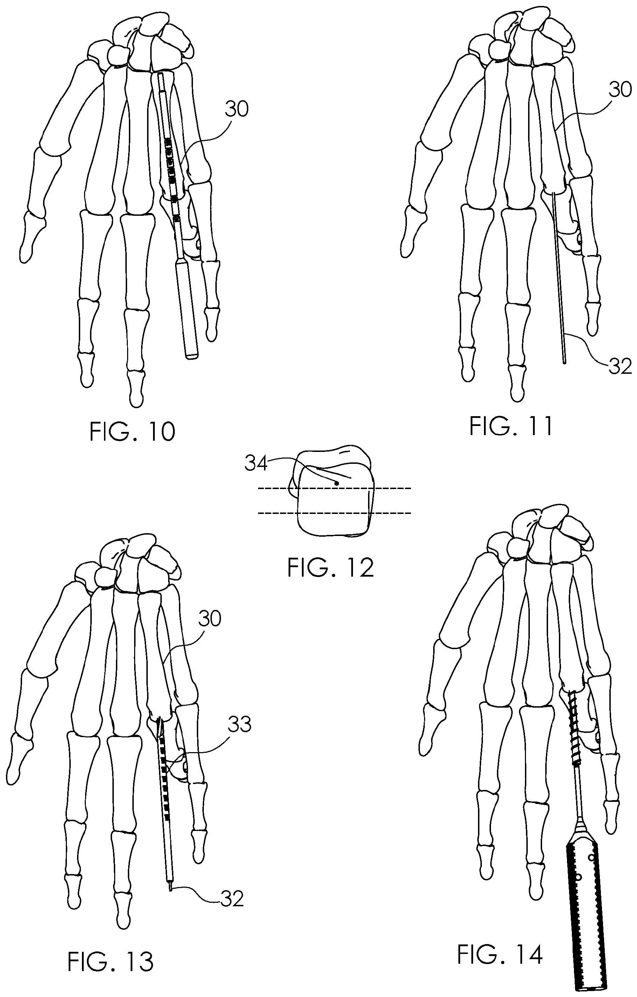

In FIG. 10, in a first step of a surgical technique which does not form part of the invention as claimed the size of the metacarpal 30 at issue is determined in order to size the implant.

In FIG. 11, it is illustrated that the fracture in the bone is aligned and then a guide wire 32 is inserted in the intramedullary canal in retrograde.

FIG. 12 illustrates the optimal position 34 for the insertion of the guide wire.

In FIG. 13 a cannulated drill 33, uses the guide wire as a guide to drill an opening into the metacarpal bone which extends through the fracture and provides enough space on each side of the fracture to properly position device 10.

In FIG. 14, the device 10 is driven into the opening in the metacarpal bone by means of the drive recess. The outer diameter of the threads 28, is slightly larger than the inner diameter of the opening in the bone. This provides bone material for threads 28 to thread into and provides a tight fit for device 10.

Having thus described some embodiments of the invention, other variations and embodiments that do not depart from the scope of the invention as defined by the appended claims will become apparent to those skilled in the art. The scope of the present invention is thus not limited to any particular embodiment, but is instead set forth in the appended claims.

## Claims

1. An intramedullary implant comprising:
a proximal end (12) and a distal end (14);
a cannulated cylindrical shaft (18) between the proximal and distal ends, the shaft having an outer surface (17);
a proximal portion of the shaft, the proximal portion extending about 15 to 30% of a length of the intramedullary implant;
a double lead thread (125);
a distal portion of the shaft comprising a cutting end (19) at the distal end, wherein the cutting end comprises a bevel and two, three, or four teeth (19a), wherein the cutting end is configured to cut through bone during drilling, and wherein the cutting end comprises 2-5 equally radially spaced longitudinal cutting flutes (23) which extend lengthwise in the outer surface and through the double lead thread of the intramedullary implant; and
an intermediate portion between the proximal portion and the distal portion;
the double lead thread (125) having a first thread (126) and a second thread (127), wherein a height of the second thread is 40% to 75% of a height of the first thread;
wherein a driver recess is formed in the proximal end;
wherein a portion of the intramedullary implant comprises one or more cutting grooves extending along a longitudinal axis of the intramedullary implant;
wherein a diameter of the distal portion is 3-5 mm and is 0.4 to 2 mm smaller than a diameter of the proximal portion;
wherein the intramedullary implant has a headless design with a uniform thread lead which avoids compression.

2. The intramedullary implant of claim 1, wherein the intramedullary implant comprises titanium.

3. The intramedullary implant of claim 1, wherein the intramedullary implant comprises stainless steel.

4. The intramedullary implant of claim 1, wherein the intramedullary implant is configured to be disposed in a scaphoid, capitellum, radial head, a metacarpal, a tarsal, a metatarsal, or an ulna.

5. The intramedullary implant of claim 1, wherein the length of the intramedullary implant is less than 130 mm.

6. The intramedullary implant of claim 1, wherein the cannulated cylindrical shaft comprises a constant diameter along its length.

## Patentansprüche

1. Intramedulläres Implantat, umfassend:
ein proximales Ende (12) und ein distales Ende (14);
einen kanülierten zylindrischen Schaft (18) zwischen dem proximalen und distalen Ende, wobei der Schaft eine Außenfläche (17) aufweist;
einen proximalen Abschnitt des Schaftes, wobei sich der proximale Abschnitt über etwa 15 bis 30 % der Länge des intramedullären Implantats erstreckt;
ein Doppelganggewinde (125);
wobei ein distaler Abschnitt des Schaftes ein Schneidende (19) am distalen Ende umfasst, wobei das Schneidende eine Fase und zwei, drei oder vier Zähne (19a) umfasst, wobei das Schneidende so konfiguriert ist, dass es bei Bohren Knochen durchtrennt, und wobei das Schneidende 2 bis 5 gleichmäßig radial beabstandete Längsschneidnuten (23) umfasst, die sich in Längsrichtung auf der Außenfläche und durch das Doppelganggewinde des intramedullären Implantats erstrecken; und
einen Zwischenabschnitt zwischen dem proximalen Abschnitt und dem distalen Abschnitt;
wobei das Doppelganggewinde (125) ein erstes Gewinde (126) und ein zweites Gewinde (127) aufweist,
wobei eine Höhe des zweiten Gewindes 40 % bis 75 % einer Höhe des ersten Gewindes beträgt;
wobei im proximalen Ende eine Treiberaussparung gebildet ist;
wobei ein Abschnitt des intramedullären Implantats eine oder mehrere Schneidnuten umfasst, die sich entlang einer Längsachse des intramedullären Implantats erstrecken;
wobei ein Durchmesser des distalen Abschnitts 3-5 mm beträgt und um 0,4 bis 2 mm kleiner ist als ein Durchmesser des proximalen Abschnitts;
wobei das intramedulläre Implantat eine kopflose Konstruktion mit einer gleichmäßigen Gewindesteigung aufweist, die Kompression vermeidet.

2. Intramedulläres Implantat nach Anspruch 1, wobei das intramedulläre Implantat Titan umfasst.

3. Intramedulläres Implantat nach Anspruch 1, wobei das intramedulläre Implantat Edelstahl umfasst.

4. Intramedulläres Implantat nach Anspruch 1, wobei das intramedulläre Implantat so konfiguriert ist, dass es in einem Kahnbein, Capitulum, Radiuskopf, einem Mittelhandknochen, einem Fußwurzelknochen, Mittelfußknochen oder einer Elle angeordnet werden kann.

5. Intramedulläres Implantat nach Anspruch 1, wobei die Länge des intramedullären Implantats weniger als 130 mm beträgt.

6. Intramedulläres Implantat nach Anspruch 1, wobei der kanülierte zylindrische Schaft entlang seiner Länge einen konstanten Durchmesser umfasst.

## Revendications

1. Implant intramédullaire comprenant :
une extrémité proximale (12) et une extrémité distale (14) ;
une tige cylindrique canulée (18) entre les extrémités proximale et distale, la tige comportant une surface extérieure (17) ;
une portion proximale de la tige, la portion proximale s'étendant sur environ 15 à 30 % de la longueur de l'implant intramédullaire ;
un filet à double entrée (125) ;
une portion distale de la tige comprenant une extrémité de coupe (19) à l'extrémité distale, dans lequel cette extrémité de coupe comprend un biseau et deux, trois ou quatre dents (19a), dans lequel l'extrémité de coupe est configurée pour couper l'os pendant le forage, et dans lequel l'extrémité de coupe comprend 2-5 cannelures de coupe longitudinales équidistantes radialement (23) qui s'étendent longitudinalement dans la surface extérieure et à travers le filet à double entrée de l'implant intramédullaire ; et
une portion intermédiaire entre la portion proximale et la portion distale ;
le filet à double entrée (125) comportant un premier filet (126) et un second filet (127),
dans lequel une hauteur du second filet représente 40 % à 75 % de la hauteur du premier filet ;
dans lequel un logement d'entraînement est formé dans l'extrémité proximale ;
dans lequel une portion de l'implant intramédullaire comprend une ou plusieurs rainures de coupe s'étendant le long d'un axe longitudinal de l'implant intramédullaire ;
dans lequel la portion distale présente un diamètre de 3-5 mm qui est de 0,4 à 2 mm inférieur à celui de la portion proximale ;
dans lequel l'implant intramédullaire présente une conception sans tête avec un pas de filetage uniforme qui évite la compression.

2. Implant intramédullaire selon la revendication 1, dans lequel l'implant intramédullaire comprend du titane.

3. Implant intramédullaire selon la revendication 1, dans lequel l'implant intramédullaire comprend de l'acier inoxydable.

4. Implant intramédullaire selon la revendication 1, dans lequel l'implant intramédullaire est configuré pour être disposé dans un scaphoïde, un capitellum, une tête radiale, un métacarpien, un tarse, un métatarsien ou un ulna.

5. Implant intramédullaire selon la revendication 1, dans lequel la longueur de l'implant intramédullaire est inférieure à 130 mm.

6. Implant intramédullaire selon la revendication 1, dans lequel la tige cylindrique canulée présente un diamètre constant sur toute sa longueur.
